# EUROPEAN PATENT SPECIFICATION

(11) **EP 0 800 862 B1**
(45) Date of publication and mention of the grant of the patent: **27.02.2002**
(21) Application number: 95939386.9
(22) Date of filing: 04.12.1995
(51) Int. Cl.: B01J 20/26, B01D 15/00, A61M 1/36, C07K 1/14, B01J 47/00

(54) **ADSORBENT FOR ENDOTOXIN, TUMOR NECROSIS FACTOR-ALPHA OR INTERLEUKINS, METHOD FOR REMOVAL VIA ADSORPTION, AND ADSORBER**
ADSORBENT FÜR ENDOTOXINE, TUMOR NEKROSE FAKTOR-ALPHA ODER INTERLEUKINE, METHODE ZUR ENTFERNUNG MITTELS ADSORPTION SOWIE ADSORBER
ADSORBANT POUR LES ENDOTOXINES, LE FACTEUR ALPHA DE NECROSE TUMORALE OU LES INTERLEUKINES, PROCEDE D'ELIMINATION PAR ADSORPTION ET ADSORBEUR

(30) Priority: 26.12.1994 JP 32330894; 26.12.1994 JP 32330994; 16.02.1995 JP 2841895; 06.09.1995 JP 22929895
(43) Date of publication of application: 15.10.1997
(73) Proprietor: KANEGAFUCHI KAGAKU KOGYO KABUSHIKI KAISHA, Kita-ku Osaka-shi Osaka-fu 530 (JP)
(72) Inventor: HIRAI, Fumiyasu, Hyogo-ken 661 (JP); TANI, Nobutaka, Osaka 545 (JP); YASUDA, Takamune, Hyogo-ken 651-21 (JP); ASAHI, Takashi, Hyogo-ken 655 (JP)
(74) Representative: Mallalieu, Catherine Louise
(86) International application number: JP9502500
(87) International publication number: WO9620042

(56) References cited:
- JP-A- 3 016 639
- JP-A- 6 023 279
- JP-A- 57 081 829
- JP-A- 63 122 463
- US-A- 3 715 345
- US-A- 4 140 652
- PATENT ABSTRACTS OF JAPAN vol. 018, no. 334 (C-1216), 24 June 1994 & JP 06 079104 A (KURITA WATER IND LTD), 22 March 1994,

## Description

### TECHNICAL FIELD

The present invention involves an adsorbent for at least one substance selected from the group consisting of endotoxin, tumor necrosis factor-α (TNF-α), and interleukins (including interleukin-1β (IL-1β), interleukin-2 (IL-2), interleukin-6 (IL-6), and interleukin-8 (IL-8)), a process for adsorbing and removing the same, and an adsorber for the same.

### BACKGROUND ART

An endotoxin which exists on the surface of a Gram-negative bacterium is known as a representative substance which causes an inflammation due to bacterial infection. Production of various cytokines and/or activated complements is promoted by the action of endotoxin. Actually, substances responsible for the inflammation by bacterial infection are these cytokines and activated complements. TNF-α is regarded as the most important substance among these cytokines.

For example, in the case of septicemia, bacterial infection induces endotoxin production and causes systemic inflammatory response. When this inflammation symptom grows worse, shock symptom (septic shock) is caused, and grave conditions such as organopathy (organ insufficiency) and multi-organopathy may further be caused.

The septicemia is also caused by Gram-positive bacteria. However, in this case, the cytokines such as TNF-α and the like are produced even without endotoxin. It is considered that these cytokines are produced at the infected sites by various stimulations.

In the case of septicemia, the prognosis for patients who have a high concentration of TNF-α in their blood is not good. Therefore, TNF-α concentration reflects the gravity of the condition of the disease (Shuchuchiryo, 6, No.2, pages 115-123 (1994)).

Examples of methods for treatment of septicemia include administration of antibiotics as a countermeasure against the infection, administration of γ-globulin to activate resistance against the infection and the like. However, the mortality rate is still high. Therefore, from a medical point of view, it is desired to remove endotoxin, which is a causal agent of septicemia, and TNF-α, a cytokine which causes inflammation, from body fluid.

Furthermore, for diseases other than septicemia in which TNF-α is involved, it is known that in inflammatory bowel disease (IBD) for which the causal agent is assumed to be the abnormality of immunity, systemic lupus erythematosus (SLE), or Kawasaki disease, the concentration of TNF-α in blood is high in consistent with the serious condition of the disease and that in rheumatoid arthritis (RA) the concentration of TNF-α in synovia increases (Nipponrinsyo, 48, extra number, pages 304-311, (1990)). Therefore, methods for treatment of these diseases also may include removing TNF-α from body fluid.

It is known that adsorbents are used for removing endotoxin. For example, Japanese Patent Publication No. 1-16389 discloses a material in which polymyxin (which is a known antidote for endotoxin) is fixed to an appropriate carrier. Japanese Patent Publication No. 3-35974 discloses an adsorbent in which basic nitrogen functional groups and polymyxin are bound to vinyl polymer. Using this, endotoxin is decomposed by poiymyxin and the degradation product is adsorbed by the nitrogen functional groups. However, several steps are required to prepare such an adsorbent, and polymyxin (which is used as an antidote) is very expensive. Japanese Laid-Open Patent Publication No. 6-211900 discloses an adsorption material comprising carriers (for example, porous glass, silica gel coated with porous organic polymer, cross-linked carbohydrates and the like) having an appropriate pore size and molecular exclusion range to which a polyanion polymer is bound. However, their effective adsorption of endotoxin is small.

An adsorbent for removing TNF-α from body fluid is also known. For example, Japanese Laid-Open Patent Publication No. 6-211900 discloses an adsorption material comprising porous carriers to which the functional groups of a polyanionic chain are bound via covalent bonds. In the examples therein, a porous carrier to which dextran sulfate is bound is used. However, the interaction between the material to which dextran sulfate is bound and TNF-α is weak. Thus, its effect is merely to retard elution and the material can not adsorb TNF-α. Therefore, this adsorption material is not suitable for practical use.

As mentioned above, cytokines are very important proteinous substances as biophylactic factors which are involved in various antigen-specific or non-specific immunological inflammatory responses. Cytokines are necessary and indispensable for maintaining biological homeostasis and are produced excessively in a case of a disease which accompanies inflammation, and are involved in the formation and the prolongation of pathology of the disease.

Interleukins are known as cytokines other than TNF-α.

IL-1β is an inflammatory cytokine which is produced mainly by a monocyte and/or a macrophage upon stimulation by foreign substances such as bacteria. In 1984, human IL-1β gene was cloned by Auron et al. As can be seen from the fact that IL-1β had been designated as an endogenous pyrogen (EP), a leukocytic endogenous mediator (LEM), a lymphocyte activation factor (LAF), a B cell-activating factor (BAF), and the like until the standardized designation interleukin 1 (IL-1) was determined at the 2nd International Lymphokine Workshop in 1979, IL-1β has many biological activities. Though IL-1β, which is one of the main mediators of inflammation, plays an important role in various reactions including homeostasis of an organism in a normal state, it has become clear that IL-1β induces the destruction of tissues or formation and/or deterioration of pathology in a patient with inflammatory disease when IL-1β is produced excessively or continuously for a long term by some mechanism (Biomedica, 9, 1993, pages 703-707). Particularly, it is suggested that IL-1β is involved in pathological conditions such as toxic syndrome including septicemia, RA, Lyme disease, osteoporosis, Kawasaki disease, gout, glomerulonephritis, dilated cardiomyopathy, endometritis, premature labor, granuloma, acute myelogenous leukemia, Alzheimer's disease, Down's syndrome, hepatic fibrosis, cirrhosis, alcoholic hepatitis and the like (Nipponigakukan, Cytokine -From Foundation to The Latest Information-, 1991, pages 13-20 and pages 177-187). Thus, a method for specifically suppressing IL-1β has been desired and studied enthusiastically. As representatives, an anti-IL-1β antibody, an anti-IL-1β receptor antibody, an IL-1 receptor antagonist (IL-1ra) and the like were developed (Igakunoayumi, 167, 1993, pages 432-435) and some of them were subjected to clinical trials for septicemia as an objective disease. However, none of them yielded an effect as expected and have yet to be put to practical use.

IL-2 is a glycoprotein having a molecular weight of approximately 15 kDa and was formerly called T cell growth factor (TCGF). The gene for IL-2 was cloned by Taniguchi et al. in 1983. It is intended to apply IL-2 as a therapeutic agent for cancer by using IL-2 alone or for local adoptive immunotherapy, since IL-2 has a growth promoting activity on T cells.

Alternatively, a finding has been obtained from an experiment using a transgenic mouse, which suggests that IL-2 continuously produced at a local site (e.g., at pancreas) is one potent causal agent of autoimmune diabetes mellitus (Health W. R., Allison J. et al., Nature, 359. page 547, 1992). It was also reported that an administration of IL-2 caused a development of systemic lupus erythematosus (SLE) and/or RA, both of which are autoimmune diseases (Chazerain P., Meyer O., Kahn M.-F. et al., Ann. Intern. Med., 116, page 427, 1992 ; and Wandl U. B., Nagel-Hiemke M., May D. et al. Clin. Immunol. Immunopathol., 65, page 70, 1992). Furthermore, it is suggested that IL-2 together with TNF-α is involved in pathologic conditions in septic shock (Endo S., Inada K., Inoue Y. et al. : Circulatory Shock, 38, page 264, 1992). As mentioned above, the side effects from IL-2 are serious. Therefore, a method for suppressing the action of IL-2 is desired. At present, however, no effective method for suppressing the action of IL-2 in these diseases has yet been established.

IL-6 was originally isolated as a differentiating factor for B cells. The structure of the gene for IL-6 was determined by Hirano and Kishimoto et al. in 1986. It is recognized that IL-6 functions as one of main mediators of inflammation, for example, to function as a growth factor for myelocytoma, and to induce an acute phase protein in the liver. It is shown that B cells are polyclonally activated upon abnormal production of IL-6 and then plasmacytoma is caused by producing a transgenic mouse which produces an abnormal level of IL-6. In general, it has been observed that the concentration of IL-6 in blood is high in many patients with acute inflammatory diseases, bacterial infection, virus infection, scald, myocardial infarction and the like. The IL-6 level increases at the time of invasion such as scald or surgical operation. A case has been reported wherein up to 500 ng/ml of IL-6 was detected in a cerebrospinal fluid from a patient with an acute bacterial meningitis (by pneumococcus, staphylococcus, or Listeria). IL-6 has also been detected in local inflammatory tissues or systemically in patients with chronic inflammation. In patients with autoimmune diseases such as RA, Castleman's disease or atrial myxoma, an abnormal production of IL-6 has been observed and it is considered that this triggers the production of proteins in hypergammaglobulinemia. In addition, it is suggested that IL-6 is involved in diseases such as cancer of the uterine cervix, AIDS, alcoholic hepatitis, multiple myeloma, Lennert's T lymphoma, mesangial proliferative nephritis, renal cytoma, psoriasis and septicemia (Nipponigakukan, Cytokine -From Foundation to The Latest Information-, 1991, pages 177-187). Thus, an excess amount of IL-6 is not preferred in a body. At present, however, no effective method for specifically suppressing the action of IL-6 in patients with these diseases has been established.

IL-8 was purified as a monocyte derived neutrophil chemotactic factor (MDNCF) and the gene for IL-8 was also cloned by Matsushima et al. in 1987. IL-8 is a neutrophil-activating migration regulatory factor produced by various types of cells. An infiltration of neutrophils and lymphocytes upon administering IL-8 interdermally, subcutaneously or in the arthrosis has been observed in vivo. A continuous administration of IL-8 in a large amount is very harmful to tissues, and causes destruction of tissues like those observed with adult respiratory distress syndrome in an alveolus, and destruction of tissues accompanying infiltration of lymphocytes in a large amount into an arthrosis. Experimentally, it is shown that IL-8 is involved essentially in dermatitis induced by lipopolysaccharide (LPS) and an infiltration of neutrophils during reperfusion after ischemia. This has been proved by the fact that destruction of tissues accompanying the above-mentioned infiltration of lymphocytes or neutrophils can be inhibited almost completely by administering a neutralizing antibody against IL-8. Furthermore, an abnormally high concentration of IL-8 has been detected at inflammatory sites in or in peripheral blood from patients with diseases such as RA, gouty arthritis, psoriasis, contact dermatitis, septicemia, idiopathic pulumonary fibrosis, adult respiratory distress syndrome, IBD, immune angiitis, glomerulonephritis, urinary tract infection, myocardial infarction, asthma, respiratory tract infection, perinatal infectious disease and rejection in organ transplantation, compared with those of normal individuals (Menekiyakuri, 12, No. 1, pages 15-21, 1994). Thus, IL-8 is considered to be involved in these diseases. Therefore, an excess amount of IL-8 is not preferred in a body. At present, however, no effective method for suppressing an action of IL-8 in patients with these diseases has been established.

### DISCLOSURE OF THE INVENTION

As a result of the extensive investigation on an appropriate carrier to remove above-mentioned endotoxin, TNF-α and/or interleukins, the inventors found that styrene-divinylbenzene copolymer having sulfonic acid groups was effective for such a removal. Based on this finding, the inventors obtained the present invention.

The present invention is set forth in the appended claims.

### BRIEF DESCRIPTION OF THE DRAWING

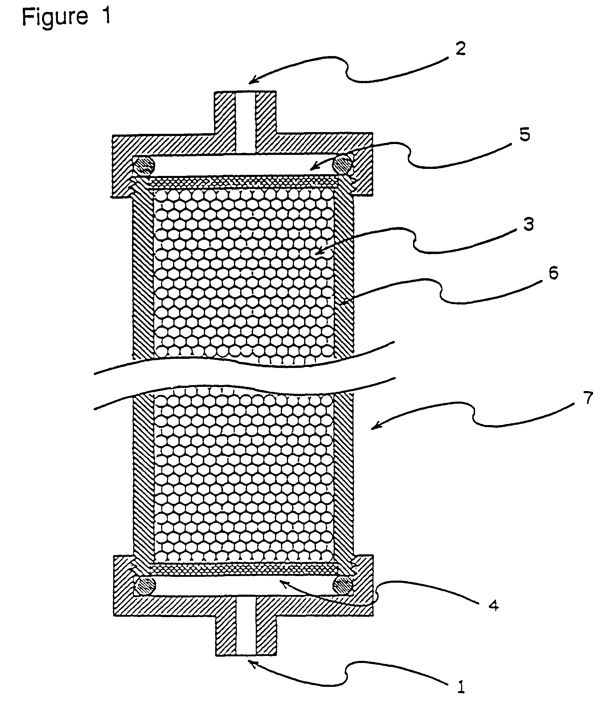

Figure 1 is a schematic cross sectional view of one exemplary adsorber for endotoxin, TNF-α and/or interleukins of the present invention. The symbols in the figure indicate, 1: an inlet or an outlet for a fluid; 2: an inlet or an outlet for a fluid; 3: an adsorbent for endotoxin, TNF-α, and/or interleukins; 4 and 5: a filter; 6: a column, and 7: a vessel, respectively.

### BEST MODE FOR CARRYING OUT THE INVENTION

As used herein, a solution means a fluid containing endotoxin, TNF-α and/or interleukins, which includes a body fluid, a culture fluid and the like. A body fluid includes blood, plasma, serum, ascites, lymph, synovia, a fractionated component therefrom, and other liquid components derived from an organism. A culture fluid refers to a culture suspension in which endotoxin and/or microorganisms producing endotoxin are contaminated, or a culture suspension of cells producing TNF-α or interleukins, which also includes a supernatant of a culture suspension, or disrupted cells, for example. For example, a culture fluid may be a supernatant of a culture suspension, disrupted cells or supernatant thereof obtained by culturing cells producing TNF-α or recombinant cells containing TNF-α gene. A culture fluid may also be a supernatant of a culture suspension, disrupted cells or supernatant thereof obtained by culturing cells producing interleukins or recombinant cells containing genes for interleukins.

Endotoxin is a substance also called lipopolysaccharide or pyrogen, and composed of a polysaccharide portion and a lipid portion (with is called lipid A) which is attached covalently to a polysaccharide portion through 2-keto-3-deoxyoctonic acid. It is considered that the endotoxin activity is present in the lipid A portion.

TNF-α is a simple protein of 157 amino acids having a molecular weight of 17 kDa, and generally exists as a trimer.

IL-1β is a protein of 153 amino acids having a molecular weight of 17,500 whose isoelectric point is 7 to 8. IL-1β is produced by a macrophage and a monocyte and has various biological activities such as induction of proliferation or differentiation of an immunor te, an endogenous pyrogen activity, and induction of inflammatory response (for example, synthesis of acute phase inflammatory proteins in the liver).

IL-2 is a glycoprotein having a molecular weight of approximately 15,000 and is produced by a T cell. It is known that IL-2 promotes proliferation, differentiation or functional activation of a T cell, a B cell, a NK cell, a monocyte, or a macrophage, for example.

IL-6 is a glycoprotein having a molecular weight of 21 to 28 kDa and is produced by a non-lymphoid cell as well as by a lymphoid cell.

IL-8 is a protein of 72 amino acids having a molecular weight of approximately 8,000, and is produced by a macrophage, a fibroblast and a vascular endothelial cell.

As used herein, the term interleukins means substances selected from IL-1β, IL-2, IL-6 and IL-8.

Styrene-divinylbenzene copolymer having sulfonic acid groups of the present invention is generally used as a strong acidic cation exchange resin. It can be in a form of, without limitation, particles, a board, a film, or fibers, for example.

In case where the adsorbent is in the form of particles, it is not preferable that it is in the form of fine particles, and it is preferable that the diameter of the particles is 200 µm or more. More preferably, it may be used under a condition that particles which are either too small or too big are excluded, that is, under the condition that the distribution of the particle size is restricted and the average size of the particles is 200 µm to 1,000 µm. If the average size of the particles is less than 200 µm, the flow of the fluid may become insufficient.

In case where the adsorbent is in a form of hollow fibers, the adsorbent is preferably used for body fluid. It is preferable that the inner diameter of the hollow fibers is 5 µm or more. If the inner diameter is less than 5 µm, cells in the body fluid may not pass smoothly. In case where the adsorbent is in a form of fibers and is packed (not hollow), it is preferable that the diameter is 1 µm or more. If the diameter is less than 1 µm, cells in the body fluid may be adsorbed non-specifically.

In case where the adsorbent is used being charged in a column and is used for body fluid, it is preferable that enough open spaces are made such that cells in body fluid can pass smoothly through them . In case where the adsorbent is used for a culture fluid, it is preferable that the supernatant from which cells or residue of the cells are removed is used.

In order to avoid non-specific adsorption of blood cell components while blood passes through the adsorbent, for example, the adsorbent may be coated with an appropriate macromolecule such as a polymer of hydroxyethylmethacrylate. This coating can also prevent the adsorbent from generating fine particles.

There are various copolymerization methods to obtain the styrene-divinylbenzene copolymer of the present invention, and any of the methods can be used. Typically, a method in which an appropriate amount of divinylbenzene is added to styrene, a polymerization catalyst (for example small amount of benzoyl peroxide and water) is added, a suspension agent such as bentonite and alginic acid is added, and the mixture is stirred vigorously to allow polymerization, can be used.

There are various kinds of methods for introducing sulfonic acid groups into styrene-divinylbenzene copolymer of the present invention. For example, the method includes, but not limited to, treating above-mentioned copolymer with concentrated sulfuric acid or chlorosulfonic acid.

The amount of the sulfonic acid groups introduced to styrene-divinylbenzene copolymer can be expressed as the ion exchange capacity. Sulfonic acid groups should be introduced into the copolymer in an appropriate density such that endotoxin, TNF-α and/or interleukins are adsorbed. It is desirable that sulfonic acid groups are introduced into the copolymer in order to ensure that predominant proteins are not adsorbed non-specifically. The ion exchange capacity of the adsorbent of the present invention is preferably 0.01 to 5 meq/ml, more preferably 0.1 to 2 meq/ml. It is desirable that the ion exchange capacity is not less than 0.01 meq/ml since the ability of the adsorbent to adsorb endotoxin, TNF-α and/or the interleukins may be lowered. On the other hand, in case where the ion exchange capacity is more than 5 meq/ml, it is difficult to generate an adsorbent which maintains the ability to adsorb endotoxin, TNF-α and/or interleukins.

The adsorbent of the present invention can adsorb endotoxin, TNF-α and/or interleukins even only on its outer surface. It is desirable that the adsorbent has pores on its surface, such that more endotoxin, TNF-α and/or interleukins are adsorbed, wherein the size of the pores are enough for endotoxin, TNF-α and/or interleukins to enter inside. The pore size varies and the distribution can be measured by means of a mercury porosimetry method or a nitrogen adsorption method. In order to adsorb endotoxin, TNF-α and/or interleukins, the main distribution of the pore size is preferably 25 to 2,000 Å, more preferably 50 to 2000 Å and even more preferably 100 to 1000 Å.

Although the adsorbent of the present invention can, as described above, adsorb endotoxin, TNF-α and/or interleukins even only on its outer surface, it is desirable that the area of the surface per one adsorbent for adsorption (specific surface area), is larger for adsorbing more endotoxin, TNF-α and/or interleukins. The specific surface area is preferably 10 m²/g or more, more preferably 100 m²/g or more.

There are various kinds of methods for absorbing and removing endotoxin, TNF-α and/or interleukins in a fluid comprising contacting the styrene-divinylbenzene copolymer having sulfonic acid groups with the fluid. The typical methods are as followings; a method which comprises taking out the fluid, storing it in a bag, for example, mixing it with the adsorbent to remove endotoxin, TNF-α and/or interleukins, and removing the adsorbent by filtration to obtain a fluid free of endotoxin, TNF-α and/or interleukins; a method in which the fluid is passed through a vessel in which the adsorbent is charged, wherein the vessel has an inlet and an outlet for the fluid and is equipped with a filter at the outlet through which the fluid can pass preventing the adsorbent from flowing out, for example. Although either of the methods can be used, the latter method is suitable for the adsorbent of the present invention since it is easy to operate and is capable of effectively removing endotoxin, TNF-α and/or interleukins from the fluid from patients using on-line system in which the vessel is adapted to incorporate an extracorporeal circulation circuit.

Next, the adsorber for endotoxin, TNF-α and/or interleukins of the present invention is explained based on a schematic cross sectional view of Figure 1. However, the adsorber of the present invention is not limited to this example.

The vessel 7 shown in Figure 1 has 1: an inlet or an outlet for a fluid, 2: an outlet or an inlet for a fluid, 3: the adsorbent for endotoxin, TNF-α and/or interleukins of the present invention, 4 and 5: means to prevent the adsorbent from flowing out, through which the fluid and the component contained in the fluid can pass preventing the adsorbent for endotoxin, TNF-α and/or the interleukins from flowing out, and 6: a column. The form and material of the vessel are not specifically limited. However, for example, a cylindrical vessel having capacity of approximately 150 to 400 ml and a diameter of approximately 4 to 10 cm is preferably used.

Hereinafter, the present invention will be specifically described by way of examples. However, the invention is not limited to the examples.

### (Example 1)

A strong acidic cation-exchange resin Diaion HPK-55H (MITSUBISHI KASEI CORPORATION, a styrene-divinylbenzene copolymer having sulfonic acid groups and having ion exchange capacity of about 1 meq/ml) was converted into Na-type, and equilibrated with physiological saline. Then, 0.5 ml of this ion-exchange resin was placed into a test tube and excess physiological saline was removed. 3 ml of human serum containing about 1000 pg/ml of endotoxin was added to the resin, and the mixture was shaken at 37°C for 2 hours. After treating the supernatant with perchloric acid, the concentration of the endotoxin in the supernatant was determined by using the limulus test with chromogenic substrate (SEIKAGAKU KOGYO CO., LTD.).

### (Comparative Example 1)

0.5 ml of physiological saline was placed into a test tube, 3 ml of human serum containing about 1000 pg/ml of endotoxin was added to the saline, and the mixture was shaken at 37°C for 2 hours. The concentration of endotoxin in the supernatant was determined as described in Example 1.

### <Results>

| | Concentration of endotoxin in the supernatant |
|---|---|
| Example 1 | 290 pg/ml |
| Comparative Example 1 | 790 pg/ml |

It was found that the concentration of endotoxin in Example 1 was greatly lowered compared with that in Comparative Example 1.

### (Example 2)

0.5 ml of ion-exchange resin obtained as described in Example 1 was placed into a test tube, and excess physiological saline was removed. 3 ml of human serum containing about 20 ng/ml of TNF-α was added to the resin, and the mixture was shaken at 37°C for 2 hours. The concentration of TNF-α in the supernatant was measured by means of ELISA method.

### (Comparative Example 2)

0.5 ml of physiological saline was placed into a test tube, 3 ml of human serum containing about 20 ng/ml of TNF-α was added to the saline, and the mixture was shaken at 37°C for 2 hours. The concentration of TNF-α in the supernatant was measured by means of ELISA method.

### <Results>

| | Concentration of TNF-α in the supernatant |
|---|---|
| Example 2 | 6.8 ng/ml |
| Comparative Example 2 | 21 ng/ml |

It was found that the concentration of TNF-α in Example 2 was greatly lowered compared with that in Comparative Example 2.

### (Example 3)

0.5 ml of ion-exchange resin obtained as described in Example 1 was placed into a test tube, and excess physiological saline was removed. 3 ml of human serum containing about 1.3 ng/ml of IL-1β was added to the resin, and the mixture was shaken at 37°C for 2 hours. The concentration of IL-1β in the supernatant was measured by means of ELISA method.

### (Comparative Example 3)

0.5 ml of physiological saline was placed into a test tube, 3 ml of human serum containing about 1.3 ng/ml of IL-1β was added to the saline, and the mixture was shaken at 37°C for 2 hours. The concentration of IL-1β in the supernatant was measured by means of ELISA method.

### <Results>

| | Concentration of IL-1β in the supernatant |
|---|---|
| Example 3 | 0.4 ng/ml |
| Comparative Example 3 | 1.1 ng/ml |

It was found that the concentration of IL-1β in the supernatant in Example 3 is greatly lowered compared with that in Comparative Example 3, and that IL-1β in the fluid could be efficiently adsorbed and removed by using the above-mentioned strong acidic cation-exchange resin.

### (Example 4)

0.5 ml of ion-exchange resin obtained in as described in Example 1 was placed into a test tube, and excess physiological saline was removed. 3 ml of human serum containing about 750 pg/ml of IL-2 was added to the resin, and the mixture was shaken at 37°C for 2 hours. The concentration of IL-2 in the supernatant was measured by means of ELISA method.

### (Comparative Example 4)

0.5 ml of physiological saline was placed into a test tube, 3 ml of human serum containing about 750 pg/ml of IL-2 was added to the saline, and the mixture was shaken at 37°C for 2 hours. The concentration of IL-2 in the supernatant was measured by means of ELISA method.

### <Results>

| | Concentration of IL-2 in the supernatant |
|---|---|
| Example 4 | 151 pg/ml |
| Comparative Example 4 | 705 pg/ml |

It was found that the concentration of IL-2 in the supernatant in Example 4 was greatly lowered compared with that in Comparative Example 4, and that IL-2 in the fluid could be efficiently adsorbed and removed by using the above-mentioned strong acidic cation-exchange resin.

### (Example 5)

0.5 ml of ion-exchange resin obtained as described in Example 1 was placed into a test tube, and excess physiological saline was removed. 3 ml of human serum containing about 420 pg/ml of IL-6 was added to the resin, and the mixture was shaken at 37°C for 2 hours. The concentration of IL-6 in the supernatant was measured by means of ELISA method.

### (Comparative Example 5)

0.5 ml of physiological saline was placed into a test tube, 3 ml of human serum containing about 420 pg/ml of IL-6 was added to the saline, and the mixture was shaken at 37°C for 2 hours. The concentration of IL-6 in the supernatant was measured by means of ELISA method.

### <Results>

| | Concentration of IL-6 in the supernatant |
|---|---|
| Example 5 | 100 pg/ml |
| Comparative Example 5 | 360 pg/ml |

It was found that the concentration of IL-6 in the supernatant in Example 5 was greatly lowered compared with that in Comparative Example 5, and that IL-6 in the fluid could be efficiently adsorbed and removed by using the above-mentioned strong acidic cation-exchange resin.

### (Example 6)

0.5 ml of ion-exchange resin obtained as described in Example 1 was placed into a test tube, and excess physiological saline was removed. 3 ml of human serum containing about 7.4 ng/ml of IL-8 was added to the resin, and the mixture was shaken at 37°C for 2 hours. The concentration of IL-8 in the supernatant was measured by means of ELISA method.

### (Comparative Example 6)

0.5 ml of physiological saline was placed into a test tube, 3 ml of human serum containing about 7.4 ng/ml of IL-8 was added to the saline, and the mixture was shaken at 37°C for 2 hours. The concentration of IL-8 in the supernatant was measured by means of ELISA method.

### <Results>

| | Concentration of IL-8 in the supernatant |
|---|---|
| Example 6 | 0.2 ng/ml |
| Comparative Example 6 | 6.3 ng/ml |

It was found that the concentration of IL-8 in the supernatant in Example 6 was greatly lowered compared with that in Comparative Example 6, and that IL-8 in the fluid could be efficiently adsorbed and removed by using the above-mentioned strong acidic cation-exchange resin.

### INDUSTRIAL APPLICABILITY

The adsorbent involved in the present invention comprising styrene-divinylbenzene copolymer having sulfonic acid groups can be used to efficiently adsorb and remove endotoxin, TNF-α and/or interleukins in a fluid. The present invention can provide an effective method to suppress the action of endotoxin, TNF-α and/or interleukins in patients with various diseases of which the causal agents are endotoxin, TNF-α and/or interleukins.

## Claims

1. Use of an adsorbent comprising a styrene-divinylbenzene copolymer having sulfonic acid groups in the preparation of a medicament for the treatment of a disease of which the causal agent is at least one substance selected from endotoxin, tumor necrosis factor-α, interleukin-1β, interleukin-2, interleukin-6 and interleukin-8.

2. Use as claimed in claim 1, wherein the ion exchange capacity of said styrene-divinylbenzene copolymer having sulfonic acid groups is between 0.01 meq/ml and 5 meq/ml.

3. Use as claimed in claim 1 or claim 2, wherein said adsorbent is charged in a vessel having an inlet and an outlet for the fluid.

4. Use as claimed in any one of claims 1 to 3, wherein said disease is an inflammatory disease.

5. Use as claimed in claim 3, wherein said vessel is adapted to incorporate an extracorporcal circulation circuit.

6. An ex-vivo method for removing at least one substance selected from endotoxing, tumor necrosis factor-α, interleukin-1β, interleukin-2, interleukin-6 and interleukin-8, wherein the method comprises contacting an adsorbent comprising styrene-divinylbenzene copolymer having sulfonic acid groups with a fluid which contains at least one substance selected from endotoxin, tumor necrosis factor-α, interleukin-1β, interleukin-2, interleukin-6 and interleukin-8,

7. The ex-vivo method as claimed in claim 6, wherein the ion exchange capacity of said styrene-divinylbenzene copolymer having sulfonic acid groups is between 0.01 meq/ml and 5 meq/ml.

8. The ex-vivo method as claimed in claim 6 or claim 7, wherein said adsorbent is charged in a vessel having an inlet and an outlet for the fluid.

9. Use of an adsorbent comprising a styrene-divinylbenzene copolymer having sulfonic acid groups in the manufacture of a vessel having an inlet and an outlet for a fluid for use in removing at least one substance selected from the group consisting of from endotoxin, tumor necrosis factor-α, interleukin-1β, interleukin-2, interleukin-6 and interleukin-8 from the fluid.

## Patentansprüche

1. Verwendung von Adsorbens, das Styrol/Divinylbenzol-Copolymer mit Sulfonsäuregruppen umfasst, zur Herstellung eines Medikaments zur Behandlung einer Krankheit, bei der das verursachende Agens mindestens eine Substanz ausgewählt aus Endotoxin, Tumornekrosefaktor-α, Interleukin-1β, Interleukin-2, Interleukin-6 und Interleukin-8 ist.

2. Verwendung nach Anspruch 1, bei der die Ionenaustauschkapazität des Styrol/Divinylbenzol-Copolymers mit Sulfonsäuregruppen zwischen 0,01 mÄq/ml und 6 mÄq/ml beträgt.

3. Verwendung nach Anspruch 1 oder Anspruch 2, bei der das Adsorbens in ein Gefäß mit einem Einlass und einem Auslass für die Flüssigkeit eingebracht wird.

4. Verwendung nach einem der Ansprüche 1 bis 3, bei der die Krankheit eine entzündliche Krankheit ist.

5. Verwendung nach Anspruch 3, bei der das Gefäß zur Einbeziehung eines extrakorporalen Kreislaufsystems ausgebildet ist.

6. Ex-vivo-Verfahren zur Entfernung von mindestens einer Substanz ausgewählt aus Endotoxin, Tumornekrosefaktor-α, Interleukin-1β, Interleukin-2, Interleukin-6 und Interleukin-8, bei dem Adsorbens, das Styrol/Divinylbenzol-Copolymer mit Sulfonsäuregruppen umfasst, mit Flüssigkeit kontaktiert wird, die mindestens eine Substanz ausgewählt aus Endotoxin, Tumornekrosefaktor-α, Interleukin-1β, Interleukin-2, Interleukin-6 und Interleukin-8 enthält.

7. Ex-vivo-Verfahren nach Anspruch 6, bei dem die Ionenaustauschkapazität des Styrol/Divinylbenzol-Copolymers mit Sulfonsäuregruppen zwischen 0,01 mÄq/ml und 5 mÄq/ml liegt.

8. Ex-vivo-Verfahren nach Anspruch 6 oder Anspruch 7, bei dem das Adsorbens in ein Gefäß mit einem Einlass und einem Auslass für die Flüssigkeit eingebracht wird.

9. Verwendung von Adsorbens, das Styrol/Divinylbenzol-Copolymer mit Sulfonsäuregruppen umfasst, zur Herstellung eines Gefäßes mit einem Einlass und einem Auslass für Flüssigkeit zur Verwendung zur Entfernung von mindestens einer Substanz ausgewählt aus Endotoxin, Tumornekrosefaktor-α, Interleukin-1β, Interleukin-2, Interleukin-6 und Interleukin-8.

## Revendications

1. Utilisation d'un adsorbant comprenant un copolymère styrène/divinylbenzène ayant des groupes acide sulfonique dans la préparation d'un médicament destiné au traitement d'une maladie dont l'agent causal est au moins une substance choisie parmi l'endotoxine, le facteur α de nécrose tumorale, l'interleukine-1β, l'interleukine-2, l'interleukine-6 et l'interleukine-8.

2. Utilisation selon la revendication 1, dans laquelle la capacité d'échange d'ions dudit copolymère styrène/divinylbenzène ayant des groupes acide sulfonique est comprise entre 0,01 méq/ml et 5 méq/ml.

3. Utilisation selon la revendication 1 ou 2, dans laquelle ledit adsorbant est chargé dans un récipient ayant une entrée et une sortie pour le fluide.

4. Utilisation selon l'une quelconque des revendi-cations 1 à 3, dans laquelle ladite maladie est une maladie inflammatoire.

5. Utilisation selon la revendication 3, dans laquelle ledit récipient est adapté pour incorporer un circuit de circulation extracorporel.

6. Procédé *ex vivo* pour éliminer au moins une substance choisie parmi l'endotoxine, le facteur α de nécrose tumorale, l'interleukine-1β, l'interleukine-2, l'interleukine-6 et l'interleukine-8, dans lequel le procédé comprend la mise en contact d'un adsorbant comprenant un copolymère styrène/divinylbenzène ayant des groupes acide sulfonique avec un fluide qui contient au moins une substance choisie parmi l'endotoxine, le facteur α de nécrose tumorale, l'interleukine-1β, l'interleukine-2, l'interleukine-6 et l'interleukine-8.

7. Procédé *ex vivo* selon la revendication 6, dans lequel la capacité d'échange d'ions dudit copolymère styrène/divinylbenzène ayant des groupes acide sulfonique est comprise entre 0,01 méq/ml et 5 méq/ml.

8. Procédé *ex vivo* selon la revendication 6 ou 7, dans lequel ledit adsorbant est chargé dans un récipient ayant une entrée et une sortie pour le fluide.

9. Utilisation d'un adsorbant comprenant un copolymère styrène/divinylbenzène ayant des groupes acide sulfonique dans la fabrication d'un récipient ayant une entrée et une sortie pour un fluide pour une utilisation dans l'élimination d'au moins une substance choisie dans le groupe formé de l'endotoxine, le facteur α de nécrose tumorale, l'interleukine-1β, l'interleukine-2, l'inter-leukine-6 et l'interleukine-8, d'avec le fluide.
